(19) Europäisches Patentamt
European Patent Office
Office européen des brevets

(11) Veröffentlichungsnummer: **0 264 751**
**A2**

# (12) EUROPÄISCHE PATENTANMELDUNG

(21) Anmeldenummer: **87114815.1**

(22) Anmeldetag: **10.10.87**

(51) Int. Cl.⁴: **C07C 65/105**, C08G 61/02, B41M 5/22

(30) Priorität: **21.10.86 DE 3635742**

(43) Veröffentlichungstag der Anmeldung: **27.04.88 Patentblatt 88/17**

(84) Benannte Vertragsstaaten: **DE FR GB**

(71) Anmelder: **BAYER AG**
**Konzernverwaltung RP Patentabteilung**
**D-5090 Leverkusen 1 Bayerwerk(DE)**

(72) Erfinder: **Scholl, Thomas, Dr.**
**Witzfelderstrasse 47a**
**D-4005 Meerbusch(DE)**
Erfinder: **Jabs, Gert, Dr.**
**Wingensiefer Kamp 25**
**D-5068 Odenthal(DE)**
Erfinder: **Richartz, Adolf, Dr.**
**No. 6, German School Lane**
**Beachland Apapa(NG)**

## (54) Hydroxycarbonsäurederivate und ihre Verwendung in Aufzeichnungsmaterialien.

(57) Hydroxycarbonsäurederivate der Formel

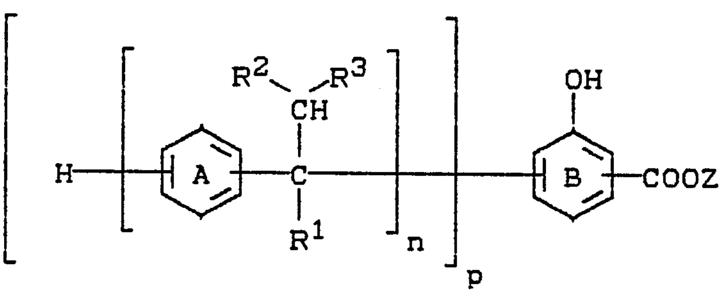

(I)

eignen sich insbesondere als Farbentwickler in Aufzeichnungsmaterialien.

EP 0 264 751 A2

# Hydroxycarbonsäurederivate und ihre Verwendung in Aufzeichnungsmaterialien

Die vorliegende Erfindung betrifft Hydroxycarbonsäurederivate und ihre Verwendung in druck-oder wärmeempfindlichen Aufzeichnungsmaterialien (Reaktionsaufzeichnungssystemen).

Reaktionsaufzeichnungssysteme im Sinne der Erfindung sind insbesondere Papiere, auf denen durch bildmäßigen mechanischen Druck oder durch bildmäßiges Erhitzen sichtbare Darstellungen erzeugt werden können.

Hierzu gehören die bekannten Reaktionsdurchschreibepapiere (vgl. M. Gutcho, Capsule Technology and Microencapsulation, Noyes Data Corporation, 1972, Seiten 242-277; G. Baxter in Microencapsulation, Processes and Applications, herausgegeben von J.E. Vandegaer, Plenum Press New York, London, Seiten 127-143).

Reaktionsdurchschreibepapiere bestehen beispielsweise aus zwei oder mehreren lose aufeinandergelegten Papierblättern, wobei das jeweils obere auf der Rückseite eine Geberschicht und das jeweils untere auf der Vorderseite eine Nehmerschicht enthält. Es ist also jeweils eine Geberschicht und eine Nehmerschicht miteinander in Kontakt. Die Geberschicht enthält z.B. Mikrokapseln, deren Kernmaterial eine Lösung eines Farbstoffbildners in einem organischen Lösungsmittel ist und die Nehmerschicht enthält einen Farbentwickler, d.h. ein Material, das den Farbstoffbildner zum Farbstoff umwandelt. Eine Durchschrift entsteht, wenn die Mikrokapseln durch den Druck eines Schreibgerätes zerstört werden und der Farbstoffbildner mit dem Farbentwickler bildmäßig reagiert.

Wenn der Farbstoffbildner in einem schmelzbaren Wachs eingebettet ist statt in Mikrokapseln, entsteht eine Durchschrift, wenn das Papier bildmäßig erhitzt wird. Es handelt sich dann um ein thermoreaktives Aufzeichnungssystem.

Farbstoffbildner und Farbentwickler können auch auf demselben Papierblatt aufgebracht sein. Man spricht dann von "self contained paper". Auf solchem Material kann durch bildmäßigen Druck bzw. bildmäßiges Erhitzen z.B. eine Schrift erzeugt werden.

Thermoreaktive Aufzeichnungssysteme werden bevorzugt in elektronischen Rechnern, Ferndruckern, Fernschreibern und Meßinstrumenten verwendet. Man kann auf ihnen Markierungen auch mit Hilfe von Laserstrahlen erzeugen.

Thermoreaktive Aufzeichnungssysteme können so aufgebaut sein, daß der Farbstoffbildner in einer Bindemittelschicht gelöst oder dispergiert ist und in einer zweiten Schicht der Farbentwickler in dem selben Bindemittel gelöst oder dispergiert ist. Farbstoffbildner und Farbentwickler können aber auch in einer Schicht dispergiert werden. Das Bindemittel wird durch Wärme erweicht und kommt an den Stellen, an denen Wärme angewendet wird, mit dem Farbentwickler in Kontakt. Dabei entwickelt sich die Farbe.

Typische Beispiele für bekannte Farbentwickler sind Aktivton-Substanzen, wie Attapulgus-Ton, Säureton, Bentonit oder Montmorillonit; ferner Halloysit, Zeolith, Siliciumdioxid, Aluminiumoxid, Aluminiumsulfat, Aluminiumphosphat, Zinkchlorid, Kaolin sowie andere Tone oder sauer reagierende organische Verbindungen, wie z.B. ringsubstituierte Phenole, Salicylsäure, deren Metallsalze oder Salicylsäureester, ferner sauer reagierende polymere Materialien, wie phenolische Polymerisate, Alkylphenolacetylenharze, Maleinsäure-Kolophonium-Harz oder teilweise oder vollständig hydrolysierte Polymerisate aus Maleinsäureanhydrid und Styrol, Ethylen oder Vinylmethylether oder Polyacetale.

Die Farbentwickler können zusätzlich auch im Gemisch mit an sich unreaktiven oder wenig reaktiven Pigmenten oder weiteren Hilfsstoffen wie Kieselgel eingesetzt werden. Beispiele für solche Pigmente sind: Talk, Titandioxid, Zinkoxid, Kreide; Tone wie Kaolin sowie organische Pigmente, z.B. Harnstoff-Formaldehyd-oder Melamin-Formaldehyd-Kondensationsprodukte.

Aktivierte Tone sind feuchtigkeitsempfindlich, d.h. die entwickelte Farbe läßt sich mit Wasser beseitigen oder bildet sich in einer feuchten Atmosphäre nur sehr schwach aus. Die Entwicklung schwarzer Fluoranfarbstoffe, wie z.B. 3-Diethylamino-6-methyl-7-anilinofluoran oder 3-(N-Cyclohexyl-N-methylamino)-6-methyl-7-anilinofluoran ist nicht möglich. Vielmehr ergeben sich mit entsprechenden organischen Farbbildnern rötlich-schwarze oder grünlich-schwarze Bilder, die schnell zu rotbraunen verblassen.

In der DE-A-2 242 250 sowie in den BE-PS 784 913 und 802 914 werden Metallsalze von substituierten Hydroxyarylcarbonsäuren, z.B. der Salicylsäure als Farbentwickler beschrieben. Aus der DE-A-2 348 639 sind außerdem Mischungen aus aromatischen Carbonsäuren oder von deren Salzen mit Polymeren bekannt. Die aromatischen Carbonsäuren bzw. deren Salze sind häufig in Wasser löslich, weshalb beim Auftragen der wäßrigen Druckpaste ein Teil der Säuren in das Innere des Blattes diffundiert, woraus ein geringeres Farbbildungsvermögen und damit eine niedrigere Farbdichte resultiert.

Aus der EP-A-0 181 283 sind bereits Metallsalicylate und ihre Verwendung in Aufzeichnungsmaterialien bekannt. Sie sind jedoch monomere und kristalline Verbindungen mit relativ hohem Schmelzpunkt.

Phenole als Farbentwickler werden z.B. in US-PS 3 244 550 beschrieben, Phenolharze z.B. in US 3 672 935 und schließlich der spezielle Einsatz von Bisphenol A-Harzen, z.B. in JA-PS 063 958.

Phenole und Phenolharze, die derzeit dem Stand der Technik entsprechen, weisen insbesondere folgende Nachteile auf:

-Bei der Durchschrift zeigt sich - etwa im Vergleich zu Ton-Entwicklern - eine geringere Intensität bzw. Farbstärke.

-Die Entwicklungsgeschwindigkeit der Durchschrift ist gering. Zu Beginn bleibt die Durchschrift blaß, bis sich im Laufe der Zeit allmählich die Intensität erhöht.

-Beschichtete Farbentwickler-Papiere zeigen eine ausgeprägte Vergilbungstendenz. Diese wird durch Sonnenlicht, aber auch künstliche Lichtquellen noch verstärkt.

Gegenstand der Erfindung sind Verbindungen folgender Formel (I)

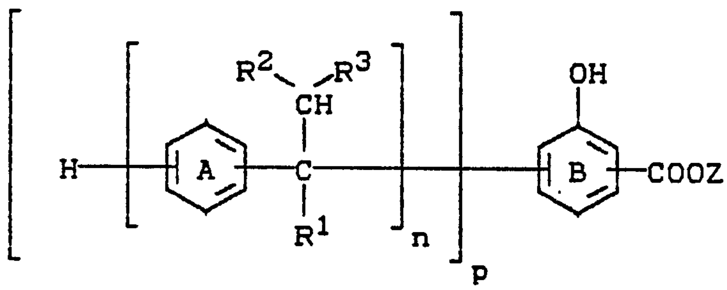

(I)

worin die Ringe A und B weitere Substituenten aufweisen können und worin bedeuten

$R^1$ bis $R^3$ unabhängig voneinander H oder Alkyl (insbesondere mit 1 bis 4 C-Atomen) oder zusammen mit wenigstens 2 C-Atomen des Ringes A den Rest zur Vervollständigung eines insbesondere carbocyclischen Ringes

Z $\frac{M^{m+}}{m}$

M m-wertiges Metallion, insbesondere $Cu^{2+}$, $Zn^{2+}$, $Fe^{2+}$, $Fe^{3+}$, $Al^{3+}$, $Mg^2$ oder $Ca^{2+}$

m ganze Zahl, insbesondere 2 oder 3

n ganze Zahl, wenigstens 2, insbesondere 2 bis 30, speziell 3 bis 6.

p ganze Zahl von 1 bis 3

In einer besonders bevorzugten Ausführungsform entsprechen die Verbindungen der oben angegebenen Formel (II) folgender Struktur

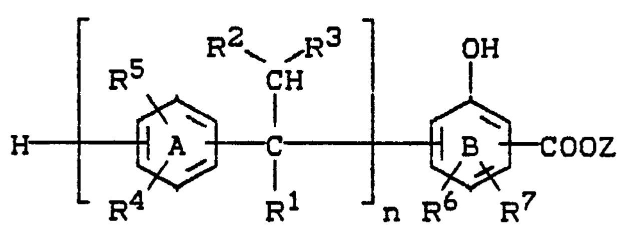

worin $R^1$ bis $R^3$, Z, M, m und n die oben angegebene Bedeutung haben und worin bedeuten

$R^4$ bis $R^7$ unabhängig voneinander Wasserstoff, Alkyl, insbesondere mit bis 1 bis 18 C-Atomen, Aralkyl, insbesondere Benzyl, Halogen, insbesondere Hydroxyl, Alkoxy, insbesondere mit 1 bis 24 C-Atomen, COOH, $-COOR^8$, CN, $NO_2$ oder $-O-CO-R^{11}$ oder Cycloalkyl, wobei $R^6$ und $R^7$ unabhängig voneinander auch

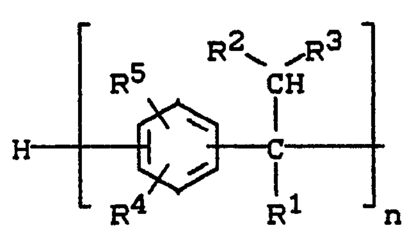

bedeuten können

$R^8$ Alkyl, insbesondere mit 1 bis 24 C-Atomen, Aryl, insbesondere Phenyl oder $NR^9R^{10}$

$R^9$, $R^{10}$ unabhängig voneinander Wasserstoff, Alkyl, insbesondere mit 1 bis 24 C-Atomen
$R^{11}$ Alkyl, insbesondere $C_1$-$C_{18}$

In einer besonders bevorzugten Ausführungsform steht die Gruppe COOZ in Ring B in o-Stellung zur OH-Gruppe.

Gegenstand der Erfindung ist weiterhin ein Verfahren zur Herstellung von harzartigen Hydroxycarbonsäuren bzw. ihren Derivaten durch Umsetzung von Verbindungen der Formel (III)

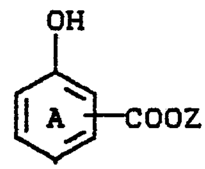

(III)

mit Verbindungen der Formel (IV)

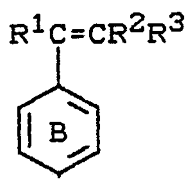

(IV)

worin die Ringe A und B substituiert sein können und Z sowie $R^1$ bis $R^3$ die oben angegebene Bedeutung haben, dadurch gekennzeichnet, daß man die Verbindungen III und IV bei Temperaturen von 50 bis 150° C in Gegenwart saurer Katalysatoren zur Reaktion bringt und anschließend mit einem Metallsalz einer aliphatischen Carbonsäure bei der selben Temperatur umsetzt.

In einer besonders bevorzugten Form des erfindungsgemäßen Verfahrens entspricht die Verbindung der Formel (III) folgender Struktur

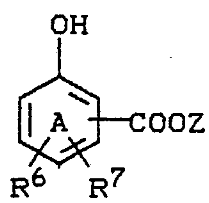

und die Verbindung der Formel (IV) folgender Struktur

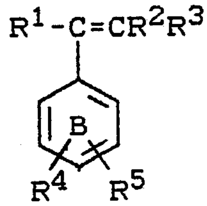

worin Z und $R^1$ bis $R^7$ die oben angegebene Bedeutung haben.

Gegenstand der Erfindung sind weiterhin Reaktionsaufzeichnungsmaterialien mit Verbindungen der Formel (I).

Gegenstand der Erfindung ist weiterhin ein Verfahren zur Herstellung von Aufzeichnungen mit einem Reaktionsaufzeichnungssystem mit Hilfe eines Farbentwicklers, bei dem der Farbentwickler der oben angegebenen Formel (I) entspricht und/oder nach dem erfindungsgemäßen Verfahren erhältlich ist.

Die erfindungsgemäßen Metallsalze von Umsetzungsprodukten aromatischer Hydroxycarbonsäuren (III) mit Vinylbenzol-Derivaten (IV) werden durch Erhitzen der Komponenten in Gegenwart saurer Katalysatoren und Zugabe von Metallsalzen bei Temperaturen von 40 bis 170° C hergestellt. Als Katalysatoren werden Brönsted-Säuren wie $H_2SO_4$ und p-Toluolsulfonsäure eingesetzt.

Die erfindungsgemäßen Produkte stellen vorzugsweise farblose bis blaßgelbe Harze mit Erweichungspunkten von 25 bis 125° C dar, Säurezahlen von 20 bis 200 und besitzen mittlere Molekulargewichte von 300 bis 3,000.

Bevorzugte Ausgangsverbindungen (III) sind Salicylsäure, p-Hydroxybenzoesäure, 3-Nonyl-salicylsäure, 5-tert.-octylsalicylsäure, 3-Methyl-5-tert.-amylsalicylsäure, 3-Methyl-5-tert.-octylsalicylsäure, 3-Cyclohexyl-5-ethylsalicylsäure, 5-Chlorsalicylsäure, 2,4-Dihydroxybenzoesäure.

Bevorzugte Ausgangsverbindungen (IV) sind Styrol, 4-Chlorstyrol, 4-Methylstyrol, 4-Hydroxystyrol, α-Methylstyrol, Inden.

Natürlich können auch Gemische aus den beschriebenen Ausgangsprodukten eingesetzt werden.

Die Reaktionskomponenten können in weiten Grenzen variiert werden. Bevorzugte Produkte ergeben sich aus Molverhältnissen (III):(IV) = 1:2-8. Die Katalysatorkonzentration kann ebenfalls in weiten Grenzen variiert werden. Um zu einheitlichen Produkten zu gelangen, ist man jedoch bestrebt, seine Konzentration möglichst niedrig zu halten (ca. 0,5 bis 3 Gew.-%, bezogen auf Verbindung (IV)).

Als Katalysatoren eignen sich Brönsted-Säuren besonders gut. Dies sind vor allem Schwefelsäure und p-Toluolsulfonsäure, Dodecylbenzolsulfonsäure, Salzsäure u.a. Der Vorteil der Brönsted-Säuren gegenüber den Lewis-Säuren wie $AlCl_3$, $BF_3$, $SnCl_4$, $TiCl_4$, mit denen die Reaktion zwar auch gelingt, ist die Helligkeit der Produkte, die für die Verwendung als Farbentwickler in Reaktionsschreibesystemen essentiell ist.

Als Metallsalze werden Übergangsmetallsalze sowie Salze der II. und III. Hauptgruppe des Periodensystems eingesetzt. Es empfiehlt sich, Metallsalze schwacher organischer Säuren einzusetzen, wie z.B. Acetate, Propionate, Butyrate etc. Bevorzugt sind Acetate des Zinks, Fe, Mg, Ca, Al. Von diesen wird das Zinksalz besonders bevorzugt.

Zur Herstellung der erfindungsgemäßen Produkte legt man zweckmäßigerweise die aromatische Hydroxycarbonsäure in reiner Form oder in einem inerten Lösungsmittel, wie Chlorbenzol, zusammen mit der Brönsted-Säure, z.B. Schwefelsäure, vor und versetzt mit der Vinylbenzol-Verbindung (IV) bei Temperaturen von 40 bis 170° C, bevorzugt 60 bis 120° C. Anschließend setzt man ein Metallsalz hinzu, bevorzugt Metallacetat, und destilliert im Falle von Metallacetaten Essigsäure ab.

Das so hergestellte Harz kann auf dieser Stufe durch Zugabe eines Lösungsmittels und Wasser gewaschen werden. Restspuren an saurem Katalysator beeinflussen die Farbentwicklungseigenschaften der Produkte nur unmerklich, so daß die Wäsche mit Wasser oft überflüssig ist. Wichtiger ist die Einhaltung der Temperaturobergrenze von max. 170° C, da darüber hinaus langsame Decarboxylierung des Harzes einsetzt, was mit Einbußen der Farbentwicklereigenschaften einhergeht.

Die erfindungsgemäßen Farbentwickler sowie die beschriebenen wäßrigen Dispersionen können beispielsweise zur Herstellung kohlefreier Durchschreibepapiere sowie zur Herstellung von thermoreaktiven Aufzeichnungsmaterialien verwendet werden. Hierzu werden die Dispersionen der erfindungsgemäßen Farbentwickler auf eine Papierträgerbahn aufgestrichen. Die Formulierung derartiger Streichfarben ist bekannt.

Eine zur Herstellung eines kohlefreien Durchschreibepapiers zu verwendende Streichfarbe kann beispielsweise durch mechanische Dispergierung des Harzpulvers in Wasser erfolgen, das genügende Mengen an Dispergiermittel enthält.

Als Dispergiermittel können z.B. Polyvinylacetate, Polyvinylalkohole, Hydroxyethylcellulose, Gummiarabicum, Guar gum, Locust bean gum oder Ghatti gum verwendet werden. Besonders bevorzugt sind Dispersionen, die neben den erfindungsgemäßen Farbentwicklern Kombinationen aus verschiedenen Polysacchariden enthalten: Gummi arabicum und Guar gum, Gummi arabicum und Locust bean gum; hierbei wirkt Gummi arabicum offenbar als Dispergiermittel, während das andere Polysaccarid als Verdicker das Sedimentieren und die Aggolmeration von Harzteilchen verhindert.

Zur mechanischen Dispergierung sind handelsübliche Kolloidmühlen, Perlmühlen, Kugelmühlen und ähnliche Homogenisiereinrichtungen geeignet.

Zur weiteren Formulierung der Streichfarben werden der wäßrigen Dispersion häufig Absorbentien wie Kreide, Streichchlay, Aluminiumsilikate etc. zugegeben.

Besonders günstig sind die Farbentwicklungseigenschaften der erfindungsgemäßen Farbentwickler, wenn sie als "Hybrid-Systeme" eingesetzt werden, d.h. wenn sie etwa mit chemisch modifizierten Aluminium-Schichtsilikaten auf Montmorrillonit-Basis kombiniert werden.

Ferner müssen die Streichfarben mit Binder versehen werden, um die Farbentwickler auf einen Träger zu fixieren. Da bevorzugt Papier als Trägerstoff infrage kommt, handelt es sich bei diesen Bindern hauptsächlich um Papierbeschichtungsmittel, wie Gummiarabikum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylmono-oder -copolymere.

Die erfindungsgemäßen Farbgeber enthaltenden Streichmassen gestatten den Einsatz verschiedener bekannter Beschichtungstechniken, beispielsweise den Auftrag mit blade-coater oder anderer gängiger Beschichtungstechniken.

Neben wäßrigen Streichfarben ist jedoch auch die Einarbeitung in Druckfarben für Flexo-oder Offsetdruck möglich.

Bei der Herstellung einer Offset-oder Buchdruckfarbe können die erfindungsgemäßen Entwicklerharze mit einem geeigneten Firnis auf einem Dreiwalzenstuhl angerieben werden. Die Herstellung derartiger Offsetdruckarben ist bekannter Stand der Technik.

Flexodruckfarben enthalten neben Bindemitteln ein leicht siedendes Lösungsmittel. Hierfür sind als Lösungsmittel z.B. $C_1$-$C_6$-Alkohole, $C_2$-$C_4$-Alkandiole, -triole, Ethylenglykolmonoalkylether, aromatische Kohlenwasserstoffe, Ester und/oder Ketone geeignet. Lösungsmittel sind ferner Methanol, Ethanol, n-Propanol, Isopropanol, n-Butanol, Isobutanol, sec.-Butanol, Ethylenglykolmonomethylether, Ethylenglykol, 1,2-Propandiol, Glycerin, Aceton, Methylethylketon, Toluol, Xylol oder Gemische davon. Die Herstellung von Flexodruckfarben gehört ebenfalls zum längst bekannten Stand der Technik.

Mit Hilfe der Druckfarben lassen sich bestimmte Zonen des Trägermaterials spotweise beispielsweise mit Offset-oder Flexodruckwerken auftragen.

Arbeitet man nach diesem Verfahren, können spotbeschichtete Entwicklerpapiere hergestellt werden und das bisher übliche, zonenweise Neutralisieren eines ganzflächig beschichteten Entwicklerpapiers an den Stellen, wo auf Sicherheitsgründen keine Durchschrift erzeugt werden soll, kann entfallen.

Die erfindungsgemäßen Entwicklerharze können ebenfalls zur Herstellung von Thermopapieren verwendet werden.

Thermoreaktive Aufzeichnungssysteme umfassen z.B. wärmeempfindliche Aufzeichnungs-und Kopiermaterialien und -papiere. Diese Systeme werden beispielsweise zum Aufzeichnen von Informationen, z.B. in elektronischen Rechnern, Ferndruckern, Fernschreibern oder in Aufzeichnungsgeräten und Messinstrumenten verwendet, wie z.B. Elektrocardiographen. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, daß der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, daß sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Entwickler in Kontakt und es entwickelt sich sofort die erwünschte Farbe.

Die Herstellung von Thermopapieren gehört zum bekannten Stand der Technik.

In den folgenden Herstellungs-und Anwendungsbeispielen beziehen sich die angegebenen Prozentsätze, wenn nichts anderes angegeben ist, auf das Gewicht und Teile sind Gewichtsteile.

## Beispiele

### Beispiel 1

Eine Mischung von 552 g Salicylsäure und 20 g $H_2SO_4$ konz. in 1200 ml Chlorbenzol wird bei ca. 50-60° C mit 1248 g Styrol versetzt. Die entstandene klare Lösung wird nach 3stündigem Nachrühren bei 130° C abgekühlt und bei 50° C mit 438 g Zn $(OAc)_2 \bullet 2\ H_2O$ versetzt. Das Lösungsmittel wird daraufhin im Vakuum vollständig abdestilliert. Man erhält 1924 g leicht gelbliches Harz mit einem Erweichungspunkt von 45° C, Säurezahl 117, OH-Zahl 194-204, mittleres Molekulargewicht 430 (nach GPC).

### Beispiel 2

Eine Mischung von 35 g Salicylsäure und 104 g p-Chlorstyrol wird bei Raumtemperatur mit 2,5 g $H_2SO_4$ konz. versetzt, woraufhin die exotherme Reaktion einsetzt. Anschließlich erhitzt man weitere 4 Stunden bei 130° C, setzt dann bei 100 ° C 27 g $Zn(OAc)_2 \bullet 2\ H_2O$ hinzu und destilliert alle flüchtigen Bestandteile im Vakuum ab. Man erhält ca. 140 g eines gelblichen Harzes mit dem Erweichungspunkt 74° C, OH-Zahl 185, mittleres Molekulargewicht 400 (nach GPC).

Beispiel 3

Analog dem Verfahren von Beispiel 2 werden 35 g Salicylsäure in Gegenwart von 2,5 g $H_2SO_4$ konz. mit 207,8 g p-Chlorstyrol und 27 g $Zn(OAc)_2 \bullet 2 H_2O$ umgesetzt. Gelbliches Harz vom Erweichungspunkt 98° C.

Beispiel 4

Eine Mischung aus 97,5 g 5-tert.-Butylsalicylsäure und 5 g $H_2SO_4$ konz. in 80 ml Chlorbenzol wird bei 50° C mit 208 g Styrol versetzt, nach 3stündigem Nachrühren bei 130° C gibt man bei 100° C 54,9 g $(Zn(OAc)_2 \bullet 2 H_2O$ hinzu und destilliert alle flüchtigen Bestandteile im Vakuum ab. Man erhält ein gräuliches transparentes Harz vom Erweichungspunkt 49° C.

Beispiel 5

Analog dem Verfahren von Beispiel 1 wird eine Mischung aus 27,6 g Salicylsäure und 2 g $H_2SO_4$ konz. in 100 ml Chlorbenzol mit 208 g Styrol und 21,9 g $Zn(OAc)_2 \bullet 2 H_2O$ umgesetzt. Farbloses weiches Harz, OH-Zahl 70.

Beispiel 6

Analog dem Verfahren von Beispiel 1 wird eine Mischung aus 3036 g Salicylsäure und 515 g Dodecylbenzolsulfonsäure in 3300 ml Chlorbenzol mit 6864 g Styrol und 2895 g $Zn(OAc)_2 \bullet 2 H_2O$ umgesetzt. Farbloses Harz vom Erweichungspunkt 40° C mit dem mittleren Molekulargewicht 400 (nach GPC).

Beispiel 7 (zur Herstellung einer Harzemulsion)

50 Teile des Harzes gemäß Beispiel 1 wurden mit 49 Teilen einer 10 %igen wässrigen Polyvinylalkohollösung (Mowiol® 26/88 der Fa. Hoechst) sowie 1 Gewichtsteil eines Formaldehydkondensates einer Naphthalinsulfonsäure in einer Kugelmühle gemahlen, bis das Harz eine Teilchengröße von ca. 2 µm besaß.

Man erhielt eine 50 % aus Harz bestehende hochviskose Dispersion, die zur Herstellung einer wässrigen Streichfarbenformulierung eingesetzt werden kann.

Beispiel 8

10,6 kg Wasser wurden vorgelegt und mit Natronlauge auf pH 9 eingestellt. Unter intensivem Rühren erfolgte die Zugabe von 2,6 kg China Clay SPS sowie 0,9 kg gefälltes Aluminiumsilikat.

Zu 8,5 kg der so hergestellten Füllstoffdispersion wird 1 kg 50 %ige Harzemulsion gemäß Beispiel 7 zugegeben. Nachdem unter Rühren die Streichfarbe homogen geworden war, wurde 320 g eines 50 %igen Styrol-Butadien-Latex zugegeben.

Beispiel 9 (Herstellung kohlefreier Durchschreibepapiere)

Mit einem 40 µm Drahtrakel wurde die Streichfarbe gemäß Beispiel 8 auf ein Streichrohpapier mit einem Flächengewicht von 45 g/m² aufgetragen. Das Beschichtungsgewicht betrug 5 - 6 g/m² trocken.

Das so hergestellte Entwicklerpapier wird mit der beschichteten Seite mit einem mit Kapseln beschichteten handelsüblichen Durchschreibepapier in Kontakt gebracht. Nach Durchschrift, beispielsweise mit einer Schreibmaschine, entsteht eine intensiv gefärbte Kopie, je nach verwendeter Type in schwarzer oder blauer Farbe.

Beispiel 10

In 6 Teilen Druckfirnis (Brillantglanzüberdrucklack der Fa. Gebr. Schmidt, Frankfurt) wurden 2,5 Teile auf unter 2 µm pulverisierten Harzes des Beispiels 2 mit einem Kneter eingerührt. Die Mischung wurde 5 mal über einen Dreiwalzenstuhl angerieben.

Die so formulierte Druckfarbe wurde mit einer Offsetmaschine (Rotaprint) auf einen Papierträger aufgetragen. Im Kontakt mit einem handelsüblichen kohlefreien Druchschreibepapier ergab sich durch Schreibdruck an den bedruckten Partien eine Kopie.

Beispiel 11

100 g Farbgeber (z. B. Kristallviolettlacton) werden mit 750 g einer 6 %igen wässrigen Lösung von Polyvinylalkohol (Moviol® 26/88 der Fa. Hoechst) in einer Kugelmühle bis zu einer Korngröße von 2 - 4 µm gemahlen.

8 Teile dieser Dispersion wurden mit 60 Teilen der Streichfarbe gemäß Beispiel 8 vermischt.

Die Mischung wird mit einem Drahtrakel (40 µm) auf ein Streichrohpapier des Flächengewichtes 52 g/m² aufgetragen. Das Beschichtungsgewicht betrug 3 und 4 g/m².

Bei Erhitzen des farblosen Papiers entsteht bei ca. 120° C eine Blauverfärbung, die bei ca. 200° C ihre volle Farbstärke erhält.

**Ansprüche**

1. Verbindungen folgender Formel (I)

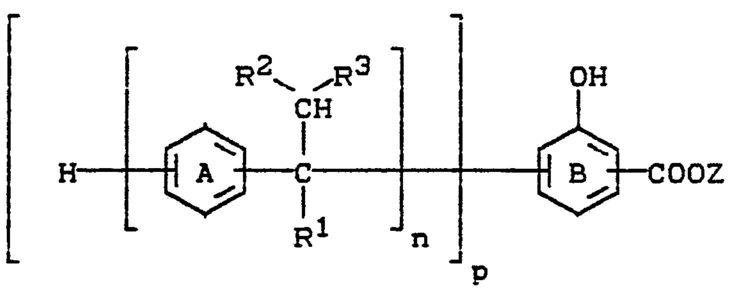

(I)

worin die Ringe A und B weitere Substituenten aufweisen können und worin bedeuten

$R^1$ bis $R^3$ unabhängig voneinander H oder Alkyl oder zusammen mit wenigstens 2 C-Atomen des Ringes A den Rest zur Vervollständigung eines Ringes,

Z $\frac{M^{m+}}{m}$

M m-wertiges Metallion,

m ganze Zahl,

n ganze Zahl, wenigstens 2,

p ganze Zahl von 1 bis 3.

2. Verbindungen gemäß Anspruch 1, dadurch gekennzeichnet, daß sie folgender Formel (II) entsprechen

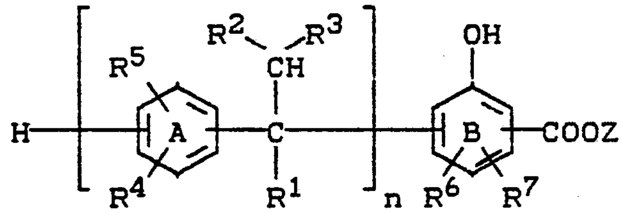

(II)

worin $R^1$ bis $R^3$, Z, M, m und n die oben angegebene Bedeutung haben und worin bedeuten

$R^4$ bis $R^7$ unabhängig voneinander Wasserstoff, Alkyl, Aralkyl, Halogen, Alkoxy, COOH, $-COOR^8$, CN, $NO_2$ oder $-O-CO-R^{11}$ oder Cycloalkyl,

wobei $R^6$ und $R^7$ auch die Bedeutung

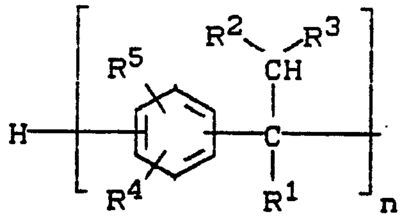

besitzen können
$R^8$ Alkyl, Aryl, insbesondere Phenyl oder $NR^9R^{10}$
$R^9$, $R^{10}$ unabhängig voneinander Wasserstoff, Alkyl,
$R^{11}$ Alkyl.

3. Verbindungen gemäß wenigstens einem der vorhergehenden Ansprüche, dadurch gekennzeichnet, daß die Gruppe COOZ in Ring B in o-Stellung zur OH-Gruppe steht.

4. Verfahren zur Herstellung von harzartigen Hydroxycarbonsäuren bzw. ihren Derivaten durch Umsetzung von Verbindungen der Formel (III)

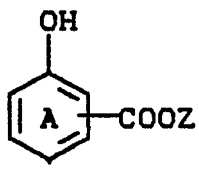

(III)

mit Verbindungen der Formel (IV)

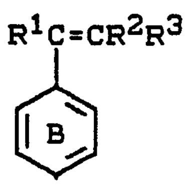

(IV)

worin die Ringe A und B substituiert sein können und Z sowie $R^1$ bis $R^3$ die oben angegebene Bedeutung haben, dadurch gekennzeichnet, daß man die Verbindungen III und IV bei Temperaturen von 50 bis 150° C in Gegenwart saurer Katalysatoren zur Reaktion bringt und anschließend mit einem Metallsalz einer aliphatischen Carbonsäure bei derselben Temperatur umsetzt.

5. Verfahren nach Anspruch 4, dadurch gekennzeichnet, daß die Verbindung der Formel (III) folgender Formel entspricht

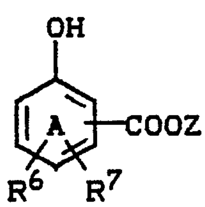

und daß die Verbindung der Formel (IV) folgender Formel entspricht

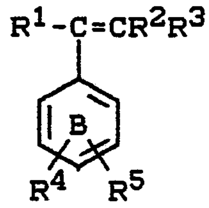

worin Z und $R^1$ bis $R^7$ die oben angegebene Bedeutung haben.

6. Reaktionsaufzeichnungssystem, dadurch gekennzeichnet, daß es wenigstens eine Verbindung gemäß Anspruch 1 enthält.

7. Verfahren zur Herstellung von Aufzeichnungen mit einem Reaktionsaufzeichnungssystem mit Hilfe eines Farbentwicklers, dadurch gekennzeichnet, daß der Farbentwickler der Formel (I) entspricht und/oder gemäß Anspruch 4 erhältlich ist.

8. Verfahren nach wenigstens einem der vorhergehenden Ansprüche, dadurch gekennzeichnet, daß M die Bedeutung $Zn^{2+}$ hat.